# EUROPÄISCHE PATENTSCHRIFT

(11) **EP 3 832 299 B1**
(45) Veröffentlichungstag und Bekanntmachung des Hinweises auf die Patenterteilung: **13.11.2024**
(21) Anmeldenummer: 19212778.5
(22) Anmeldetag: 02.12.2019
(51) Int. Cl.: G01N 27/416, G01N 33/18

(54) **VERFAHREN UND VORRICHTUNG ZUM KALIBRIEREN EINER AMPEROMETRISCHEN MESSELEKTRODE**
METHOD AND DEVICE FOR CALIBRATING AN AMPEROMETRIC MEASURING ELECTRODE
PROCÉDÉ ET DISPOSITIF D'ÉTALONNAGE D'UNE ÉLECTRODE DE MESURE AMPÉROMÉTRIQUE

(43) Veröffentlichungstag der Anmeldung: 09.06.2021
(73) Patentinhaber: Carela GmbH, 79618 Rheinfelden (DE)
(72) Erfinder: KRUMREY, Julian, 79540 Lörrach (DE); KRUMREY, Bernd, 79591 Eimeldingen (DE); REISGYS, Michael, 4132 Muttenz (CH)
(74) Vertreter: Friese Goeden Patentanwälte PartGmbB

(56) Entgegenhaltungen:
- EP-A1- 2 212 684
- JP-A- 2011 169 859
- JP-A- 2018 185 257
- US-A1- 2016 123 950
- US-A1- 2018 017 526

## Beschreibung

Die Erfindung betrifft ein Verfahren zum Kalibrieren einer amperometrischen Messelektrode für die Bestimmung eines Desinfektionsmittelgehaltes von Wasser, eine Vorrichtung insbesondere zur Durchführung dieses Verfahrens sowie die Verwendung dieser Vorrichtung zur Bestimmung des Desinfektionsmittelgehaltes von Wasser.

Die Desinfektion von Wasser ist ein wichtiger Baustein, um die Gesundheit der Menschen und Tiere in menschlicher Obhut zu erhalten und zu verbessern. Schädliche Mikroorganismen und Keime im Wasser können schwere Erkrankungen und sogar Todesfälle verursachen. Beispielsweise können nur durch das Einatmen von Legionellen-kontaminierten feinen Wassertröpfchen oder Aerosolen schwerwiegende Erkrankungen ausgelöst werden, wie das Pontiac-Fieber oder die Legionärskrankheit. Diese gefährlichen Aerosole können zum Beispiel aus kontaminierten Wasser in Duschen, aus Kühlkreisläufen über luftdurchströmte Kühltürme oder auch aus Verneblungsanlagen zur Luftbefeuchtung in Gewächshäusern freigesetzt werden.

Durch die Desinfektion des Wassers insbesondere durch chemische Desinfektionsmittel können die für Mensch und Tier gefährlichen Mikroorganismen abgetötet und unschädlich gemacht werden. Chemische Desinfektionswirkstoffe, wie beispielsweise Chlor, Natriumhypochlorit, Calciumhypochlorit, hypochlorige Säure, organisch-chemische Chlorabspalter, Chlordioxid, Brom, hypobromige Säure, organisch-chemische Bromabspalter, sind seit langem bekannt und dazu geeignet, schädliche Mikroorganismen in Wasser zu bekämpfen.

Für die Desinfektion von Trinkwasser sind insbesondere Chlor, Natriumhypochlorit, Calciumhypochlorit und Chlordioxid empfohlen und von staatlichen Behörden zugelassen. Ihr Einsatz kann gegebenenfalls von den Gesundheitsämtern angeordnet werden. Dabei sind die vorgeschriebenen gesetzlichen Grenzwerte für die Konzentration der Desinfektionswirkstoffe einzuhalten und dürfen nicht überschritten werden. Aber auch ein Unterschreiten ist problematisch, da dann die desinfizierende Wirkung möglicherweise entfällt oder abgeschwächt wird.

Aus der Praxis ist bekannt, die Konzentration des Desinfektionswirkstoffs durch die Untersuchung einer Wasserprobe mit der DPD-Methode diskontinuierlich zu bestimmen. Das DPD-Reagenz *N*,*N*-Diethyl-*p*-phenylendiamin bildet beispielsweise mit freiem Chlor eine rötliche Färbung, deren Intensität proportional zur Konzentration des freien Chlors ist und photometrisch gemessen werden kann. Das Photometer wird mit externen Farbstandards geprüft und kalibriert. Die Konzentration des Desinfektionswirkstoffs in der Probe kann somit verlässlich bestimmt werden. Die so gemessene Konzentration ist aber nur eine Momentaufnahme. Für eine zeitlich lückenlose Kontrolle der Konzentration der Desinfektionswirkstoffe im Wasser sind kontinuierlich arbeitende amperometrische Verfahren und Vorrichtungen bekannt. Diese verwenden spezifische Messelektroden, welche mit einer Auswerteelektronik ausgelesen werden.

Für verlässliche Messwerte werden die Geräte zur amperometrischen Messung direkt vor deren Einsatz und dann regelmäßig kalibriert, beispielsweise wöchentlich. Diese Kalibrierung ist nicht trivial und birgt Fehlerquellen. Standardlösungen mit festgelegten garantierten Konzentrationen der Desinfektionswirkstoffe sind schwer erhältlich und nicht lagerstabil. Sie haben sich daher in der Praxis nicht bewährt. Die Kalibrierung wird deshalb mit dem vor Ort vorhanden Desinfektionsmittel-haltigen Wasser durchgeführt. Man bestimmt in diesem Wasser mit der DPD-Methode die Konzentration der Desinfektionswirkstoffe und verwendet diesen Messwert als Normal zur Kalibrierung der amperometrischen Vorrichtung.

Bei dieser Art der Kalibrierung entstehen folgende Fehlerquellen: Durch die oftmals gepulste Zugabe des konzentrierten Desinfektionsmittels ins Wasser ändert sich die Konzentration der Desinfektionswirkstoffe an der Messelektrode. Weiterhin benötigen die üblichen Messelektroden bauartbedingt einen konstanten Volumenstrom des zu messenden Wassers, um einen zuverlässigen Messwert der Konzentration der Desinfektionswirkstoffe auszugeben. In einer ruhenden Wasserprobe, beispielsweise in einem Becherglas, ist eine Bestimmung der Konzentration der Desinfektionswirkstoffe nicht möglich oder führt aufgrund der fehlenden Strömung bei gleicher Konzentration zu abweichenden Messwerten. Dies ist problematisch, weil die amperometrischen Messelektroden in der Regel in den Leitungen einer Wasserversorgung angeordnet sind und daher bei Betrieb stets umströmt sind. Dieses Problem tritt insbesondere bei kleinen Wasserversorgungsanlagen mit geringem Durchfluss auf, wie beispielsweise Einzelversorgungen in dünnbesiedelten Landstrichen, mobile Wasserversorgungen, die Wasserversorgung auf Schiffen oder Bohrinseln sowie Wasserversorgungen in Krisengebieten, im Zivilschutz, beim Militär u. Ä. Dort sind konstante Strömungsverhältnisse und konstante Konzentrationen der Desinfektionswirkstoffe an der Messelektrode aufgrund der geringen vorhandenen Wassermenge und unregelmäßiger Wasserabnahme nur schwer zu realisieren.

JP 2011 169859 A betrifft ein automatisiertes Verfahren zur Bestimmung einer Chlorkonzentration.

Ausgehend vom Stand der Technik liegt der Erfindung somit die Aufgabe zugrunde, ein Verfahren und eine Vorrichtung zum Kalibrieren von amperometrischen Messelektroden für die Bestimmung der Konzentration von Desinfektionswirkstoffen in Wasser bereitzustellen, welches diese Fehlerquellen nicht aufweist.

Die Aufgabe wird erfindungsgemäß durch ein Verfahren gemäß Anspruch 1, eine Vorrichtung nach Anspruch 6 und eine Verwendung nach Anspruch 13 gelöst. Vorteilhafte Weiterbildungen der Erfindung finden sich in den Unteransprüchen.

Erfindungsgemäß wird ein Verfahren zum Kalibrieren einer amperometrischen Messelektrode, die zur Bestimmung der Konzentration von Desinfektionswirkstoffen in Wasser eingesetzt wird, angegeben, das sich dadurch auszeichnet, dass
(a) von einer Wasserleitung zu untersuchendendes Wasser abgezweigt und an einer amperometrischen Messelektrode vorbeigeleitet wird, wobei dieses Vorbeileiten in einem Kreislauf erfolgt,
(b) ein Messwert der amperometrischen Messelektrode bestimmt wird,
(c) die Konzentration von Desinfektionswirkstoffen des abgezweigten Wassers ermittelt wird, und
(d) die in Schritt (c) ermittelte Konzentration von Desinfektionswirkstoffen als Normal für die Kalibrierung der amperometrischen Messelektrode verwendet wird.

Mit diesem Verfahren ist es möglich, auch bei kleinen zu desinfizierenden Wassermengen, bei schwankender Konzentration von Desinfektionswirkstoffen und bei unregelmäßigen Wasserabnahmen auch zu Beginn einer Desinfektionsmaßnahme die amperometrische Messung der Konzentration von Desinfektionswirkstoffen zuverlässig zu kalibrieren.

Die Kalibrierung ist in der Messtechnik ein Messprozess zur zuverlässig reproduzierbaren Feststellung und Dokumentation der Abweichung eines Messgerätes oder einer Maßverkörperung gegenüber einem anderen Gerät oder einer anderen Maßverkörperung, die in diesem Fall als Normal bezeichnet wird. Beim erfindungsgemäßen Verfahren ist das Messgerät, das kalibriert werden soll, die amperometrische Messelektrode für die Bestimmung der Konzentration von Desinfektionswirkstoffen in Wasser. Das Normal ist im erfindungsgemäßen Verfahren die Bestimmung der Konzentration von Desinfektionswirkstoffen in Wasser mit einem anderen, diskontinuierlichen Verfahren, beispielsweise mit dem DPD-Verfahren, bei welchem das Reagenz *N*,*N*-Diethyl-*p-*phenylendiamin eingesetzt wird. Es bildet beispielsweise mit freiem Chlor (Desinfektionswirkstoff) eine rötliche Färbung, deren Intensität proportional zur Konzentration des freien Chlors ist und photometrisch gemessen werden kann. Das Photometer wird mit externen Farbstandards geprüft. Auf diese Weise kann mit einer chemischen Reaktion die Konzentration von Desinfektionswirkstoffen in Wasser bestimmt werden. Diese Bestimmung und der damit erhaltene Wert wird als Normal für die Kalibrierung eingesetzt. Mit dem in Schritt (c) bestimmten Normal kann der Messwert der amperometrischen Messelektrode mit der Konzentration von Desinfektionswirkstoffen korreliert werden. Die Erfindung wird anhand der Bestimmung des freien Chlors mit der DPD-Methode beschrieben. Selbstverständlich ist die Erfindnung nicht auf dieses Ausführungsbeispiel beschränkt. Der Fachmann wird das Prinzip leicht auf weitere Nachweismethoden oder andere Reagenzien für andere Desinfektionswirkstoffe übertragen können.

In der Definition des VIM von JCGM 2008 gehört zur Kalibrierung üblicherweise ein zweiter Schritt, nämlich die Berücksichtigung der ermittelten Abweichung bei der anschließenden Benutzung des Messgerätes zur Korrektur der abgelesenen Werte. Dieser zweite Schritt ist beim erfindungsgemäßen Verfahren optional. Die Kalibrierung kann ohne Eingriff erfolgen, der das Messgerät verändert.

Bei dem in Schritt (a) genannten zu untersuchenden Wasser handelt es sich um das Wasser, in welchem die Konzentration von Desinfektionswirkstoffen bestimmt werden soll. Im Falle einer Trinkwasserversorgung kann dieses Wasser beispielsweise unmittelbar einer Wasserleitung bzw. dem Ablauf eines Hochbehälters entnommen werden. Im ersten Betriebszustand, welcher den Normalbetrieb darstellt, kann die Konzentration von Desinfektionswirkstoffen mit der amperometrischen Messelektrode bestimmt werden und das Wasser der Hauptleitung wieder zugeführt werden. Bei kleineren Trinkwasserversorgungen kann auch der gesamte Wasserstrom über die amperometrische Messelektrode geführt werden. Im zweiten Betriebszustand, welcher den Kalibrierbetrieb darstellt, wird das entnommene Wasser in einen Kreislauf eingespeist, in dem sich die zu kalibrierende amperometrische Messelektrode befindet und dort bis zum Abschluss der Kalibrierung zirkuliert. Somit ist der Wasserkreislauf für Schritt (a) des erfindungsgemäßen Verfahrens zumindest teilweise verschieden von der Wasserleitung, dessen Wasser untersucht werden soll. Durch das Aufrechterhalten einer Strömung des zu untersuchenden Wassers im Kreislauf wird eine genaue Kalibrierung möglich, da die Abweichung der amperometrischen Messwerte einer ruhenden Wasserprobe von denen einer umströmten amperometrischen Messelektrode vermieden wird. Da andrerseits das Wasser im Kreislauf zirkuliert wird, wird sich dessen Zusammensetzung und insbesondere die Konzentration von Desinfektionswirkstoffen bis zum Abschluss der als Normal verwendeten Bestimmung der Konzentration von Desinfektionswirkstoffen mit einem anderen, diskontinuierlichen Verfahren nicht ändern. Zur Zirkulation kann eine elektrisch angetriebene Pumpe verwendet werden.

In einer Ausführungsform kann in Schritt (b) der Messwert bestimmt werden, wenn er einen etwa konstanten Wert erreicht hat. Ein solcher konstanter Wert kann ein Wert sein, bei dem der gemessene Wert nur noch um etwa ± 10 % oder etwa ± 5 % oder etwa + 3 % oder etwa ± 2 % oder etwa ± 1 % schwankt. Auf diese Weise kann sichergestellt werden, dass durch Konzentrationsschwankungen verursachte Fehler vermieden werden.

In einer Ausführungsform kann in Schritt (c) die Konzentration von Desinfektionswirkstoffen mit der N,N-Diethyl-p-phenylendiamin(DPD)-Methode bestimmt werden, wie vorstehend erläutert wurde. Die Konzentration des Desinfektionswirkstoffs in der Probe kann somit verlässlich bestimmt werden. Dieser in Schritt (c) bestimmte Wert dient als Normal, um die amperometrische Messelektrode auf der Basis des in Schritt (b) gewonnenen Messwertes zu kalibrieren.

In einer Ausführungsform kann weiterhin vom abgezweigten Wasser im Kreislauf die Temperatur und/oder der pH-Wert und/oder der Durchfluss (d.h. die Durchflussmenge des Wassers) und/oder die Trübung gemessen werden. Hierdurch kann die Bestimmung der Konzentration von Desinfektionswirkstoffen mit größerer Genauigkeit erfolgen, da der Einfluss dieser Größen auf den in Schritt (b) gewonnenen amperometrischen Messwert korrigiert werden kann.

In einer Ausführungsform können die Messwerte der amperometrischen Messelektrode und der optionalen Messung von Temperatur und/oder pH-Wert und/oder Durchfluss und/oder Trübung in einer Steuer- oder Regeleinheit verarbeitet werden. Eine solche Verarbeitung kann die Visualisierung der Messwerte umfassen, beispielsweise mit einem Display oder einer LED-Zeile mit Ampelfarben. In einigen Ausführungsformen der Erfindung können die Messwerte der Messelektrode zur Regelung der Zugabe von Desinfektionsmittel verwendet werden.

In einer Ausführungsform können die Messwerte der amperometrischen Messelektrode und optional auch die Messwerte der Messung von Temperatur und/oder pH-Wert und/oder Durchfluss und/oder Trübung in einem Datenspeicher gespeichert werden. Der Datenspeicher kann dazu mit der Steuer- oder Regeleinheit verbunden sein.

In einigen Ausführungsformen der Erfindung können die Messwerte der amperometrischen Messelektrode und optional auch die Messwerte der Messung von Temperatur und/oder pH-Wert und/oder Durchfluss mit einer Datenfernübertragungseinrichtung übertragen werden Hierzu kann die Steuer- oder Regeleinrichtung mit einer Kabelverbindung oder einer drahtlosen Verbindung mit einer räumlich abgesetzten Empfangseinheit verbunden sein.

Mit vorstehenden Bauelementen ist eine Steuerung oder eine Regelung oder/und eine Überwachung des erfindungsgemäßen Verfahrens möglich. So kann beispielsweise ein Sollwert oder ein Sollwertbereich mit Ober- und Untergrenzen festgelegt werden. Wird dieser über- oder unterschritten, kann ein Signal erzeugt werden, so dass dann beispielsweise die Desinfektionsmittelmenge geregelt werden kann.

Gegenstand der vorliegenden Erfindung ist ferner eine Vorrichtung, mit der das vorstehend beschriebene Verfahren durchgeführt werden kann. Das vorstehend beschriebene Verfahren kann dazu herangezogen werden, die Bau- und Funktionsweise der nachfolgend beschriebenen erfindungsgemäßen Vorrichtung näher zu erläutern.

Die erfindungsgemäße Vorrichtung ist eine Vorrichtung zum Kalibrieren einer amperometrischen Messelektrode, die zur Bestimmung der Konzentration von Desinfektionswirkstoffen in Wasser eingesetzt wird, wobei sie aufweist:
(i) eine Einrichtung zum Abzweigen von zu untersuchendem Wasser aus einer Leitung.
(ii) eine amperometrische Messelektrode,
(iii) Leitungen, in denen das abgezweigte Wasser im Kreislauf an der amperometrischen Messelektrode vorbei geführt wird, und
(iv) eine Entnahmevorrichtung, um einen Teil des abgezweigten Wassers aus dem Kreislauf zu entnehmen.

In einer Ausführungsform weist die Vorrichtung weiterhin zumindest einen Temperaturfühler und/oder zumindest eine pH-Messelektrode und/oder zumindest einen Durchflussmesser und/oder zumindest einen Trübungsmesser auf.

In einer Ausführungsform enthält die Einrichtung zum Abzweigen von zu untersuchendem Wasser zumindest einen Regler oder zumindest ein Ventil, beispielsweise ein Dreiwegeventil.

In einer weiteren Ausführungsform weist die Vorrichtung eine Steuer- oder Regeleinrichtung auf, in der die Messwerte der amperometrischen Messelektrode sowie gegebenenfalls des Temperaturfühlers, der pH-Messelektrode, des Trübungsmessers und/oder des Durchflussmessers verarbeitet werden.

Ferner kann in einer Ausführungsform die Vorrichtung einen Datenspeicher aufweisen, in welchem die Messwerte der amperometrischen Messelektrode sowie gegebenenfalls des Temperaturfühlers, der pH-Messelektrode, des Trübungsmessers und/oder des Durchflussmessers gespeichert werden. Weiterhin kann die Vorrichtung eine Datenfernübertragungseinrichtung aufweisen.

In einer Ausführungsform kann die Vorrichtung weiterhin einen Vorratsbehälter aufweisen. Dieser Vorratsbehälter kann sich im Kreislauf befinden, in dem das abgezweigte Wasser transportiert wird. Dazu kann an einer Stelle in den Vorratsbehälter eine Zufuhrleitung hinein und an einer anderen Stelle eine Entnahmeleitung aus dem Behälter herausführen. Die Entnahmeleitung kann mit der Saugseite einer elektrischen Pumpe gekoppelt sein, welche das entnommene Wasser im zweiten Betriebszustand, welcher den Kalibrierbetrieb darstellt, in den Kreislauf mit der amperometrischen Messelektrode einspeist und über die Zufuhrleitung in den Vorratsbehälter zurückführt. Hierdurch wird eine blasenfreie und konstante Strömung im Kreislauf ermöglicht.

Weiterhin kann die Entnahmevorrichtung mit dem Vorratsbehälter verbunden sein, so dass sichergestellt ist, dass das zur Bestimmung des Normals mit der DPD-Methode entnommene Wasser dem selben Kreislauf entstammt und die selbe Konzentration von Desinfektionswirkstoffen enthält.

Nachfolgend soll die Erfindung anhand von Figuren ohne Beschränkung des allgemeinen Erfindungsgedankens näher erläutert werden. Dabei zeigt
Fig. 1 eine Vorrichtung zur amperometrischen Messung der Konzentration von Desinfektionswirkstoffen in Wasser im ersten Betriebszustand, welcher den Normalbetrieb darstellt.
Fig. 2 zeigt die Vorrichtung gemäß Figur 1 im zweiten Betriebszustand, welcher den Kalibrierbetrieb darstellt. Anhand der Figuren 1 und 2 wird eine erfindungsgemäße Vorrichtung und das Verfahren zu deren Betrieb näher erläutert. Dabei zeigt Figur 1 den ersten Betriebszustand, welcher den Normalbetrieb darstellt und Figur 2 den zweiten Betriebszustand, welcher den Kalibrierbetrieb darstellt.

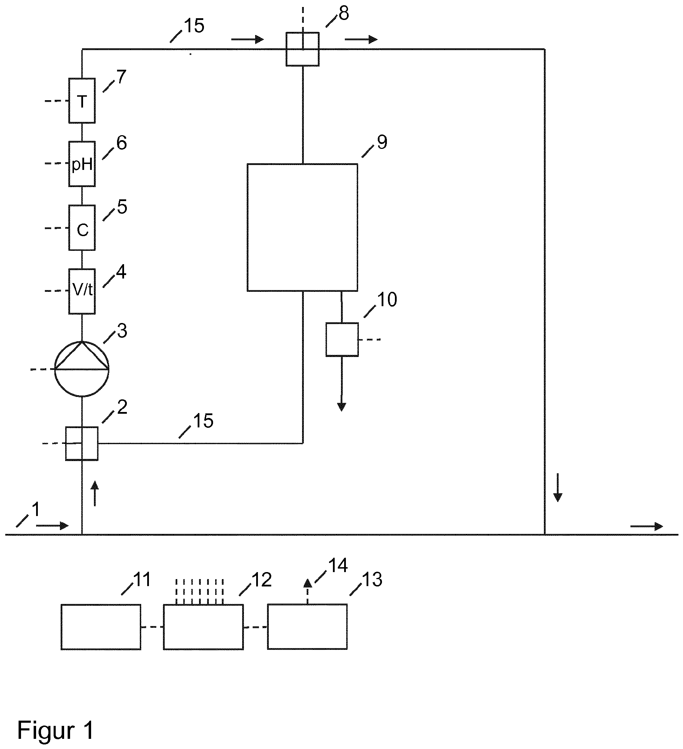

Figur 1 zeigt eine Wasserleitung 1, über welche Wasser aus einer nicht dargestellte Wasserversorgungseinrichtung, beispielsweise einem Hochbehälter oder einer Quellfassung, einem nicht dargestellten Verbraucher zugeführt werden kann, beispielsweise einem Haushalt oder einer Gemeinde. In anderen Ausführungsformen der Erfindung kann Wasser einem Schwimmbecken über die Leitung 1 entnommen werden und nach Durchlauf durch eine Filteranlage dem Schwimmbecken wieder zugeführt werden.

In der Wasserleitung 1 befindet sich eine Einrichtung 2 zum Abzweigen eines Teilstromes des zu untersuchenden Wassers. In einigen Ausführungsformen der Erfindung kann die Einrichtung 2 ein Dreiwegeventil enthalten oder daraus bestehen. Die Einrichtung 2 kann manuell oder elektrisch oder pneumatisch bedient werden. Die Einrichtung 2 kann an eine elektronische Steuer- oder Regeleinrichtung 12 angeschlossen sein, um einen vollautomatischen Betrieb des erfindungsgemäßen Verfahren zu ermöglichen. Die Einrichtung 2 zum Abzweigen eines Teilstromes ist mit der Wasserleitung 1 über ein T-Stück verbunden.

Im dargestellten Ausführungsbeispiel strömt das abgezweite Wasser hinter der Einrichtung 2 durch eine Leitung, in welcher zumindest eine amperometrische Messelektrode 5 angeordnet ist, mit welcher die Konzentration von Desinfektionswirkstoffen bestimmt wird. Optional können weitere Komponenten in dieser Leitung angeordnet sein, beispielsweise zumindest eine Pumpe 3 und/oder zumindest ein Durchflussmesser 4 und/oder zumindest eine pH-Messelektrode 6 und/oder zumindest ein Temperaturfühler 7 und/oder zumindest ein zweites Dreiwegeventil 8 und/oder zumindest ein Vorratsbehälter 9.

Der Vorratsbehälter 9 kann mit einer optionalen Entnahmevorrichtung 10 ausgestattet sein. Im dargestellten Ausführungsbeispiel ist die Entnahmevorrichtung 10 als Ablass- und Probeentnahmeventil ausgebildet. Der Vorratsbehälter 9 weist einen Einlauf auf, welcher dazu eingerichtet ist, den Vorratsbehälter 9 über einen Ausgang des zweiten Dreiwegeventils 8 zu befüllen. Weiterhin weist der Vorratsbehälter 9 einen Auslauf auf. Der Auslauf ist über eine Leitung 15 mit einem weiteren Eingang der Einrichtung 2 zum Abzweigen eines Teilstromes verbunden.

Optional kann die Vorrichtung eine Steuer- oder Regeleinrichtung 12 enthalten. Die Einrichtung 2, die amperometrische Messelektrode 5, die Pumpe 3, der Durchflussmesser 4, die pH-Messelektrode 6, der Temperaturfühler 7 und/oder das zweite Dreiwegeventil 8 können über Strom- und/oder Datenleitungen mit der Steuer- oder Regeleinrichtung 12 verbunden sein. Die elektrischen Leitungen sind in den Figuren als gestrichelte Linien dargestellt, wobei aber deren Verbindung aus Gründen der Übersichtlichkeit nicht gezeigt ist. Alternativ kann die Verbindung der Messeinrichtungen 4, 5, 6 und 7 mit der Steuer- oder Regeleinrichtung 12 auch über eine Funkverbindung erfolgen.

Die Steuer- oder Regeleinrichtung 12 kann mit einem optionalen Datenspeicher 11 und einer optionalen Datenfernübertragungseinrichtung 13 verbunden sein. In anderen Ausführungsformen der Erfindung kann die Steuer- oder Regeleinrichtung 12 einen optionalen Datenspeicher 11 und/oder eine optionale Datenfernübertragungseinrichtung 13 enthalten.

Die Datenfernübertragungseinrichtung 13 kann eine optionale Antenne 14 zur drahtlosen Datenübertragung enthalten oder Daten über ein Telefon- oder Computernetzwerk mittels einer optischen oder elektrischen Kabelverbindung übertragen. In einigen Ausführungsformen kann die erfindungsgemäße Vorrichtung auch ohne Datenfernübertragung autark betrieben werden. Vor Ort können Messwerte angezeigt, ausgelesen und Steuerbefehle eingegeben werden. Alternativ dazu kann die erfindungsgemäße Vorrichtung über die Datenfernübertragungseinrichtung 13 gesteuert, ausgelesen und in ein räumlich vernetztes Steuerungs- und Überwachungssystem eingebunden werden.

Alle Bauteile können in einem abschließbaren und manipulationssicheren Gehäuse eingebaut sein, welches in den Figuren nicht gezeigt ist.

In dem in Figur 1 gezeigten ersten Betriebszustand wird zumindest ein Teilstrom des in der Wasserleitung 1 ankommenden Wassers über das T-Stück und die Einrichtung 2 in die Leitung mit der amperometrischen Messelektrode 5 geführt. In einigen Ausführungsformen der Erfindung kann dieser Teilstrom auch die gesamte in der Wasserleitung 1 ankommende Wassermenge umfassen. Der im ersten Betriebszustand vorliegende Wasserfluss ist in Figur 1 mit Pfeilen dargestellt.

Somit wird im Normalbetrieb das abgezweigte Wasser kontinuierlich entnommen und über die amperometrische Messelektrode 5 und die weiteren, optionalen Messeinrichtungen 4, 6 und 7 geleitet. Sodann wird das abgezweigte Wasser über das zweite Dreiwegeventil 8 einer Rücklaufleitung zugeführt, welche stromabwärts des zur Entnahme verwendeten T-Stücks in der Wasserleitung 1 mündet. Der Vorratsbehälter 9 ist nicht in Betrieb und kann vorzugsweise entleert sein. Die Pumpe 3 kann zur Unterstützung des Wasserstromes über die Messeinrichtungen 4, 5, 6 und 7 verwendet werden oder aber auch bei ausreichendem Wasserdruck über eine nicht dargestellte Bypassleitung umgangen werden oder passiv durchströmt werden.

Die Messsignale des Durchflussmessers 4, der amperometrischen Messelektrode 5, der pH-Messelektrode 6 und des Temperaturfühlers 7 werden zur zentralen Steuer- oder Regeleinrichtung 12 geleitet, verarbeitet, ausgewertet, angezeigt und/oder im Datenspeicher 11 gespeichert. Über die Datenfernübertragungseinrichtung 13 mit Antenne 14 können sie auch an entfernten Orten angezeigt und bearbeitet werden. So ist eine zeitlich lückenlose Überwachung der Konzentration der Desinfektionswirkstoffe im Wasser möglich.

Bei Überschreitungen eines vorgebbaren Grenzwertes kann ein Alarm ausgelöst werden, um einen Bediener auf die Fehlfunktion der Desinfektion hinzuweisen. Alternativ oder zusätzlich kann eine nicht dargestellte Dosiereinrichtung des Desinfektionsmittels beeinflusst werden und die Zugabe reduzieren oder einstellen. Bei Unterschreitungen eines vorgebbaren Grenzwertes kann ebenfalls ein Alarm ausgelöst werden, um einen Bediener auf die Fehlfunktion der Desinfektion hinzuweisen. Alternativ oder zusätzlich kann eine nicht dargestellte Dosiereinrichtung für das Desinfektionsmittel beeinflusst werden und die Zugabe erhöht werden. Durch die kontinuierliche Überwachung kann einer Unterversorgung mit mangelhafter Desinfektionsleistung ebenso wie einer Überversorgung mit Schädigung der Wasserverbraucher wirksam vorgebeugt werden.

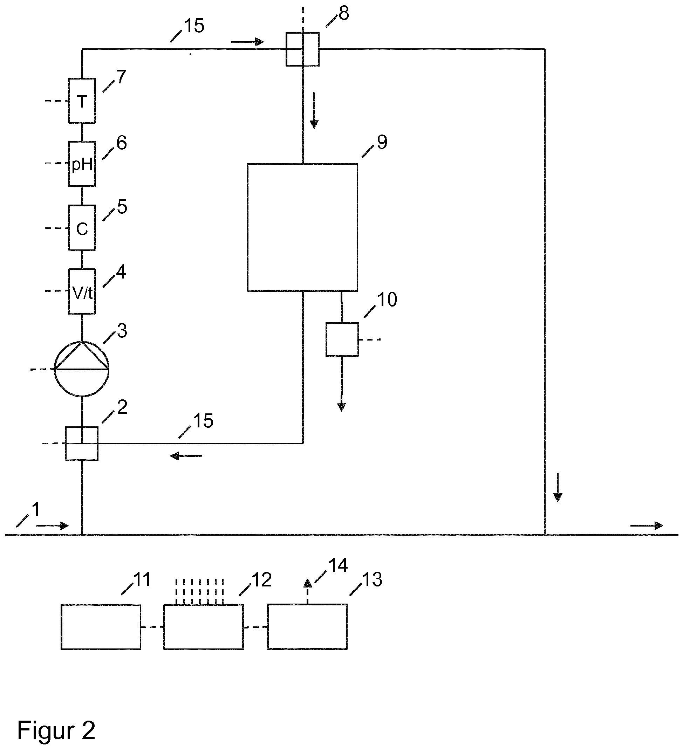

Um die amperometrischen Messelektrode 5 erstmalig oder regelmäßig im Betrieb zu kalibrieren, kann die Vorrichtung in den zweiten Betriebszustand geschlatet werden. Dieser ist in Figur 2 dargestellt. Gleiche Bestandteile der Erfindung sind mit gleichen Bezugszeichen versehen, so dass sich die nachfolgende Beschreibung auf die wesentlichen Unterschiede beschränkt. Auch in Figur 2 ist der Wasserfluss durch Pfeile symbolisiert.

Um in den zweiten Betriebszustand zu schalten, schaltet zunächst das zweite Dreiwegeventil 8, sodass die Rücklaufleitung abgetrennt und der Vorratsbehälter 9 gefüllt wird. Bei Erreichen eines vorgebbaren Füllstandes wird die Einrichtung 2 so geschaltet, dass der Ausgang des Vorratsbehälters 9 über die Leitung 15 mit dem Eingang der Pumpe 3 verbunden wird. Somit wird ein geschlossener Kreislauf abgezweigten Wassers etabliert, nämlich vom Vorratsbehälter 9 über die Pumpe 3, den Durchflussmesser 4, der amperometrischen Messelektrode 5, der pH-Messelektrode 5 und dem Temperaturfühler 7 und das zweite Dreiwegeventil 8 zurück in den Vorratsbehälter 9. Das von der Wasserversorgungseinrichtung, beispielsweise einem Hochbehälter oder einer Quellfassung, an die Verbraucher gelieferte Wasser strömt im zweiten Betriebszustand vollständig in der Wasserleitung 1 an der erfindungsgemäßen Vorrichtung vorbei.

Durch den geschlossenen Kreislauf abgezweigten Wassers ist sichergestellt, dass sich die Konzentration der Desinfektionswirkstoffe im abgezweigten Wasser nicht ändert. Wenn zumindest der Messwert der amperometrischen Messelektrode 5, vorzugsweise aber auch die Messwerte des Durchflussmessers 4, der pH-Messelektrode 6 und des Temperaturfühlers 7, konstant sind, wird die Entnahmevorrichtung 10 des Vorratsbehälters 9 geöffnet, sodass eine Probe entnommen werden kann. In dieser Probe wird die Konzentration der Desinfektionswirkstoffe mit einem alternativen, als Normal dienenden Verfahren bestimmt. Beispielsweise kann dies photometrisch mit der DPD-Methode erfolgen. Der so bestimmte Wert wird sodann zur Kalibrierung der amperometrischen Messelektrode verwendet.

Während der Bestimmung des Normals zirkuliert das abgezweigte Wasser weiter über die amperometrische Messelektrode 5, den Durchflussmesser 4, die pH-Messelektrode 6 und den Temperaturfühler 7. An der amperometrischen Messelektrode liegen dadurch konstante Werte für Strömung, Konzentration der Desinfektionswirkstoffe, pH-Wert und Temperatur an. Die Kalibrierung erfolgt so zuverlässig.

Danach wird das zweite Dreiwegeventil 8 umgeschaltet, sodass das Wasser über die Rücklaufleitung in die Wasserleitung 1 abfließen kann. Kurz darauf kann auch die Einrichtung 2 wieder umgeschaltet werden, um Wasser aus der Wasserleitung 1 zu entnehmen. Der Vorratsbehälter 9 kann entweder über die Einrichtung 2 und das Dreiwegeventil 8 in die Rücklaufleitung entleert werden oder aber über Entnahmevorrichtung 10. Das Wasser aus der Leitung 1 kann sofort weiterhin kontinuierlich überwacht werden. In einigen Ausführungsformen der Erfindung kann der vorstehend beschriebene Ablauf mehrfach wiederholt werden, um auf diese Weise eine Mehrzahl von Kalibrierpunkten bei unterschiedlichen Messwerten der Konzentration der Desinfektionswirkstoffe zu erhalten.

Selbstverständlich ist die Erfindung nicht auf die in den Figuren dargestellten Ausführungsformen beschränkt. Die vorstehende Beschreibung ist daher nicht als beschränkend, sondern als erläuternd anzusehen. Die nachfolgenden

Ansprüche sind so zu verstehen, dass ein genanntes Merkmal in zumindest einer Ausführungsform der Erfindung vorhanden ist. Dies schließt die Anwesenheit weiterer Merkmale nicht aus. Sofern die Beschreibung oder die Ansprüche ,erste' und 'zweite' Merkmale definieren, so dient dies der Unterscheidung gleichartiger Merkmale, ohne eine Rangfolge festzulegen.

## Patentansprüche

1. Verfahren zum Kalibrieren einer amperometrischen Messelektrode (5), welche zur Bestimmung der Konzentration von Desinfektionswirkstoffen in Wasser eingesetzt wird, **dadurch gekennzeichnet, dass**
(a) von einer Wasserleitung (1) zu untersuchendendes Wasser abgezweigt und an der amperometrischen Messelektrode (5) vorbeigeleitet wird, wobei dieses Vorbeileiten in einem Kreislauf erfolgt,
(b) ein Messwert der amperometrischen Messelektrode (5) bestimmt wird,
(c) die Konzentration von Desinfektionswirkstoffen des abgezweigten Wassers mit einem weiteren Verfahren ermittelt wird, und
(d) die in Schritt (c) ermittelte Konzentration von Desinfektionswirkstoffen als Normal für die Kalibrierung der amperometrischen Messelektrode (5) verwendet wird.

2. Verfahren nach Anspruch 1, **dadurch gekennzeichnet, dass** in Schritt (b) der Messwert bestimmt wird, wenn er einen etwa konstanten Wert erreicht hat.

3. Verfahren nach einem der vorhergehenden Ansprüche,
**dadurch gekennzeichnet, dass** in Schritt (c) die Konzentration von Desinfektionswirkstoffen mit der N,N-Diethyl-p-phenylendiamin(DPD)-Methode bestimmt wird.

4. Verfahren nach einem der vorhergehenden Ansprüche,
**dadurch gekennzeichnet, dass** weiterhin vom abgezweigten Wasser Temperatur und/oder pH-Wert und/oder der Durchfluss und/oder die Trübung gemessen wird.

5. Verfahren nach einem der vorhergehenden Ansprüche,
**dadurch gekennzeichnet, dass** die Messwerte der amperometrischen Messung und ggf. der Messung von Temperatur und/oder pH-Wert und/oder Durchfluss in einer Steuer- oder Regeleinheit (12) verarbeitet und/oder in einem Datenspeicher (11) gespeichert und/oder mit einer Datenfernübertragungseinrichtung (13) übertragen werden.

6. Vorrichtung zum Kalibrieren einer amperometrischen Messelektrode (5), die zur Bestimmung der Konzentration von Desinfektionswirkstoffen in Wasser eingesetzt wird, **dadurch gekennzeichnet, dass** sie umfasst:
(i) eine Einrichtung zum Abzweigen (2) von zu untersuchendem Wasser aus einer Wasserleitung (1)
(ii) eine amperometrische Messelektrode (5),
(iii) eine Leitung (15), in der das abgezweigte Wasser im Kreislauf an der amperometrischen Messelektrode (5) vorbei geführt wird, und
(iv) eine Entnahmevorrichtung (10), um einen Teil des abgezweigten Wassers zu entnehmen.

7. Vorrichtung nach Anspruch 6, **dadurch gekennzeichnet, dass** die Einrichtung zum Abzweigen (2) ein Dreiwegeventil enthält, mit welchem das abgezweigte Wasser in die Leitung (15) des Kreislaufes eingespeist wird.

8. Vorrichtung nach einem der Ansprüche 6 oder 7, **dadurch gekennzeichnet, dass** sie weiterhin zumindest einen Temperaturfühler (7) und/oder zumindest eine pH-Messelektrode (6) und/oder zumindest einen Durchflussmesser (4) und/oder zumindest einen Trübungsmesser enthält.

9. Vorrichtung nach einem der Ansprüche 6 bis 8, weiterhin enthaltend eine Steuer- oder Regeleinheit (12), in welcher die Messwerte der amperometrischen Messelektrode (5) sowie optional die Messwerte des Temperaturfühlers (7) und/oder der pH-Messsonde (6) und/oder des Durchflussmessers (4) verarbeitet werden.

10. Vorrichtung nach einem der Ansprüche 6 bis 9, weiterhin enthaltend einen Datenspeicher (11), welchem die Messwerte der amperometrischen Messelektrode (5) sowie optional die Messwerte des Temperaturfühlers (7) und/oder der pH-Messsonde (6) und/oder des Durchflussmessers (4) zugeführt werden.

11. Vorrichtung nach einem der Ansprüche 6 bis 10, weiterhin enthaltend eine Einrichtung zur Datenferübertragung (13).

12. Vorrichtung nach einem der Ansprüche 6 bis 11, weiterhin enthaltend einen Vorratsbehälter (9), in welchem sich abgezweigtes Wasser befindet und welcher mit der Entnahmevorrichtung (10) verbunden ist.

13. Verwendung der Vorrichtung nach einem der Ansprüche 6 bis 12 zum Kalibrieren einer amperometrischen Messelektrode (5), mit der die Konzentration von Desinfektionswirkstoffen von Wasser bestimmt wird.

## Claims

1. Method for calibrating an amperometric measuring electrode (5), which is used to determine the concentration of disinfecting agents in water,
**characterized in that**
(a) water from a water line (1), which shall be tested, is branched off and passed past the amperometric measuring electrode (5), this passing being carried out in a circuit,
(b) a measured value of the amperometric measuring electrode (5) is determined,
(c) the concentration of disinfecting agents of the branched water is determined by means of a further method, and
(d) the concentration of disinfecting agents that is determined in step (c) is used as a standard for the calibration of the amperometric measuring electrode (5).

2. Method according to claim 1, **characterized in that** the measured value is determined in step (b) when it has reached an approximately constant value.

3. Method according to any one of the preceding claims, **characterized in that** in step (c) the concentration of disinfecting agents is determined by means of the N,N-diethyl-p-phenylenediamine (DPD) method.

4. Method according to any one of the preceding claims, **characterized in that** the temperature and/or pH value and/or the flow rate and/or the turbidity of the branched water are also measured.

5. Method according to any one of the preceding claims, **characterized in that** the measured values of the amperometric measurement and, if applicable, the measurement of temperature and/or pH value and/or flow rate are processed in an open-loop or closed-loop control unit (12) and/or stored in a data memory (11) and/or transmitted by means of a remote data transmission apparatus (13).

6. Device for calibrating an amperometric measuring electrode (5), which is used to determine the concentration of disinfecting agents in water,
**characterized in that** it comprises:
(i) an apparatus for branching off (2) water to be tested from a water line (1),
(ii) an amperometric measuring electrode (5),
(iii) a line (15) in which the branched water is circulated past the amperometric measuring electrode (5), and
(iv) a withdrawal device (10) for withdrawing part of the branched water.

7. Device according to claim 6, **characterized in that** the apparatus for branching off (2) contains a three-way valve by means of which the branched water is fed into line (15) of the circuit.

8. Device according to any one of claims 6 and 7, **characterized in that** it also contains at least one temperature sensor (7) and/or at least one pH measuring electrode (6) and/or at least one flow meter (4) and/or at least one turbidity meter.

9. Device according to any one of claims 6 to 8, further comprising an open-loop or closed-loop control unit (12) in which the measured values of the amperometric measuring electrode (5) and optionally the measured values of the temperature sensor (7) and/or of the pH measuring probe (6) and/or of the flow meter (4) are processed.

10. Device according to any one of claims 6 to 9, further comprising a data memory (11) to which the measured values of the amperometric measuring electrode (5) and optionally the measured values of the temperature sensor (7) and/or of the pH measuring probe (6) and/or of the flow meter (4) are supplied.

11. Device according to any one of claims 6 to 10, further comprising an apparatus for remote data transmission (13).

12. Device according to any one of claims 6 to 11, further comprising a storage container (9), in which branched water is located and which is connected to the withdrawal device (10).

13. Use of the device according to any one of claims 6 to 12 for calibrating an amperometric measuring electrode (5), by means of which the concentration of disinfecting agents in water is determined.

## Revendications

1. Procédé d'étalonnage d'une électrode de mesure ampérométrique (5) utilisée pour déterminer la concentration en substances actives désinfectantes dans l'eau,
**caractérisé en ce que**
(a) on fait dériver l'eau à analyser à partir d'une conduite d'eau (1) et on la fait passer devant l'électrode de mesure ampérométrique (5), ce passage s'effectuant en circuit,
(b) on détermine une valeur de mesure de l'électrode de mesure ampérométrique (5),
(c) on détermine par un autre procédé la concentration en substances actives désinfectantes de l'eau dérivée, et
(d) on utilise la concentration en substances actives désinfectantes, déterminée à l'étape (c), comme étalon pour l'étalonnage de l'électrode de mesure ampérométrique (5).

2. Procédé selon la revendication 1,
**caractérisé en ce que**, à l'étape (b), on détermine la valeur de mesure lorsqu'elle a atteint une valeur approximativement constante.

3. Procédé selon l'une des revendications précédentes,
**caractérisé en ce que**, à l'étape (c), on détermine la concentration en substances actives désinfectantes par la méthode de la N,N-diéthyl-p-phénylènediamine (DPD).

4. Procédé selon l'une des revendications précédentes,
**caractérisé en ce que** l'on mesure en outre la température et/ou le pH et/ou le débit et/ou la turbidité de l'eau dérivée.

5. Procédé selon l'une des revendications précédentes,
**caractérisé en ce que** les valeurs de mesure de la mesure ampérométrique et, le cas échéant, de la mesure de la température et/ou du pH et/ou du débit sont traitées dans une unité de commande ou de régulation (12) et/ou sont stockées dans une mémoire de données (11) et/ou sont transmises par un moyen de télétransmission de données (13).

6. Dispositif d'étalonnage d'une électrode de mesure ampérométrique (5) utilisée pour déterminer la concentration en substances actives désinfectantes dans l'eau,
**caractérisé en ce qu'**il comprend :
(i) un moyen de dérivation (2) de l'eau à analyser à partir d'une conduite d'eau (1),
(ii) une électrode de mesure ampérométrique (5),
(iii) une conduite (15) dans laquelle l'eau dérivée est amenée en circuit devant l'électrode de mesure ampérométrique (5), et
(iv) un dispositif de prélèvement (10) pour prélever une partie de l'eau dérivée.

7. Dispositif selon la revendication 6,
**caractérisé en ce que** le moyen de dérivation (2) comprend une vanne trois voies permettant d'injecter l'eau dérivée dans la conduite (15) du circuit.

8. Dispositif selon l'une des revendications 6 ou 7,
**caractérisé en ce qu'**il comprend en outre au moins une sonde de température (7) et/ou au moins une électrode de mesure de pH (6) et/ou au moins un débitmètre (4) et/ou au moins un turbidimètre.

9. Dispositif selon l'une des revendications 6 à 8, comprenant en outre une unité de commande ou de régulation (12) dans laquelle sont traitées les valeurs de mesure de l'électrode de mesure ampérométrique (5) ainsi que, en option, les valeurs de mesure de la sonde de température (7) et/ou de la sonde de mesure de pH (6) et/ou du débitmètre (4).

10. Dispositif selon l'une des revendications 6 à 9, comprenant en outre une mémoire de données (11) à laquelle sont envoyées les valeurs de mesure de l'électrode de mesure ampérométrique (5) ainsi que, en option, les valeurs de mesure de la sonde de température (7) et/ou de la sonde de mesure de pH (6) et/ou du débitmètre (4).

11. Dispositif selon l'une des revendications 6 à 10, comprenant en outre un moyen de télétransmission de données (13).

12. Dispositif selon l'une des revendications 6 à 11, comprenant en outre un réservoir (9) dans lequel se trouve de l'eau dérivée et qui est relié au dispositif de prélèvement (10).

13. Utilisation du dispositif selon l'une des revendications 6 à 12 pour étalonner une électrode de mesure ampérométrique (5) permettant de déterminer la concentration en substances actives désinfectantes de l'eau.
